# EUROPÄISCHE PATENTANMELDUNG

(11) **EP 3 181 186 A1**
(43) Veröffentlichungstag der Anmeldung: **21.06.2017**
(21) Anmeldenummer: 16199564.2
(22) Anmeldetag: 18.11.2016
(51) Int. Cl.: A61N 1/05

(54) **ELEKTRODENIMPLANTATIONSSYSTEM**

(30) Priorität: 14.12.2015 DE 102015121726
(71) Anmelder: BIOTRONIK SE & Co. KG, 12359 Berlin (DE)
(72) Erfinder: KAISER, Dajana, 12247 Berlin (DE); JADWIZAK, Detmar, 15537 Erkner (DE); FRÜNDT, Carsten, 13057 Berlin (DE); HILLEBRAND, Gordon, 12157 Berlin (DE)
(74) Vertreter: Galander, Marcus

(57) **Zusammenfassung**

Die vorliegende Erfindung betrifft ein Elektrodenimplantationssystem, umfassend:
A) wenigstens ein längliches Implantationswerkzeug, das einen proximalen Abschnitt, der wenigstens abschnittsweise als Mandrin ausgebildet ist, und einen distalen Abschnitt, der als flexibler Führungsdraht ausgebildet ist, umfasst, wobei der proximale und der distale Abschnitt durch ein statisches oder verstellbares Anschlagelement voneinander abgegrenzt sind und das Anschlagelement eine dem distalen Abschnitt zugewandte Anschlagoberfläche umfasst; und
B) eine implantierbare Elektrode mit einer Elektrodenhülse, die einen zu der Anschlagoberfläche komplementären Anschlagbereich aufweist, der dem proximalen Abschnitt zugewandt ist.

Die Elektrodenhülse und das längliche Implantationswerkzeug sind wenigstens abschnittsweise koaxial miteinander angeordnet und entlang sowie um eine gemeinsamen Längsachse relativ zueinander verlagerbar. Weitere Aspekte der Erfindung betreffen ein längliches Implantationswerkzeug und eine implantierbare Elektrode.

## Beschreibung

Die vorliegende Erfindung betrifft ein Elektrodenimplantationssystem, ein längliches Implantationswerkzeug und eine implantierbare Elektrode.

Herz-Kreislauf-Erkrankungen zählen zu den schwerwiegendsten Krankheiten der modernen Gesellschaft. Viele Erkrankungen verlaufen auch heute noch tödlich. Vor allem ältere Menschen sind von Herz-Kreislauf-Erkrankungen betroffen. Angesichts der steigenden Lebenserwartung und der wachsenden Anzahl chronischer Herzerkrankungen ist deshalb mit einer weiteren Zunahme dieser Krankheiten zu rechnen. Immer wieder angeführte Hauptursachen sind verbreitete Einflussfaktoren einer modernen und global vernetzten Gesellschaft wie Stress, Rauchen, zu fettes Essen, damit einhergehendes Übergewicht oder Bluthochdruck. Aber auch genetische Disposition oder Virusinfektionen können Herzerkrankungen verursachen. Da sich Störungen des Herz-Kreislauf-Systems bereits in jüngeren Jahren manifestieren und besonders jüngere Menschen von Beginn an in einer diese Einflussfaktoren begünstigenden Umwelt aufwachsen, ist mit einer weiteren Verstärkung des Trends zu Herz-Kreislauf-Erkrankungen zu rechnen. Einen wichtigen Ansatzpunkt stellt deshalb die konsequente Verbesserung der medizinischen Versorgung dar.

Dies führt zum einen zu steigenden Kosten und zum anderen auch zu höheren Anforderungen an die Sicherheit medizintechnischer Produkte, zum Beispiel bei Herzschrittmachern, da zukünftig tendenziell eine zunehmende Anzahl dieser Systeme im Umlauf sein wird und die Sicherheit gegen Störungen entsprechend groß sein muss.

Durch eine inhomogene Kontraktion der einzelnen Areale der linken Herzkammer kann eine Herzschwäche, auch Herzinsuffizienz genannt, entstehen. Hauptgrund für die inhomogene, beziehungsweise asynchrone Kontraktion ist eine Störung im Reizleitungssystem des Herzens. Durch die sogenannte Cardiac Resynchronization Therapy, kurz CRT-Stimulation, wird die Kontraktion der linken Herzkammer wieder homogen, vereinfacht ausgedrückt resynchronisiert, und die Pumpkraft des Herzens kann sich wieder erholen. Das Verfahren sieht in der Regel die Verwendung von zwei Elektroden vor. Eine Elektrode wird in die rechte Herzkammer implantiert. Die zweite Elektrode wird einem Seitenarm des Coronarsinus der linken Herzkammer verankert und liegt somit auf der Außenseite der linken Herzkammer. Durch die simultane Impulsabgabe über beide Elektroden wird der linke Teil des Herzens wieder synchron elektrisch erregt und eine homogene Kontraktion ist möglich.

Eine Möglichkeit, die Elektrode an ihren Zielort zu bringen, ist die sogenannte "Over-The-Wire-Technik", kurz OTW-Technik. Die Elektrode wird dabei über einen Führungsdraht, auch "Guide Wire" genannt, bis in die gewünschte Zielvene geführt. Der Führungsdraht wird dabei in die Elektrode hinein und durch diese hindurchgeschoben. Das Einführen des Führungsdrahtes in die Elektrode kann aus distaler Richtung oder aus proximaler Richtung erfolgen. Mit dem Führungsdraht wird dann ein Weg durch die einzelnen Gefäße in die Zielvene gesucht und die Elektrode über den Führungsdraht nachgeführt. Nach Erreichen der Zielvene muss die Elektrode in dem Gefäß sicher fixiert werden. Dies wird im Falle der CRT-Elektroden gewöhnlich durch passive Fixierungselemente der Elektrode ermöglicht. Diese können in Form von äußeren, weichen Konturen, wie beispielsweise Schraubengewinden oder Noppen ausgebildet sein. Sie können aber auch als angeformte distale Bereiche, wie beispielsweise J-Kurven, S-Kurven oder ankerartige Strukturen ausgebildet sein. Die äußeren, weichen Konturen ermöglichen ein Anstemmen und Fixieren der Elektrode in der sogenannten Wedge-Position. Die angeformten distalen Bereiche erlauben eine Positionierung der Elektrode in größeren Gefäßen, indem sich die ausgebildeten Kurven oder ankerartigen Strukturen an die Gefäßwand anstemmen und somit die Elektrode in Position halten. Zu diesem Zweck wird konventionell der Führungsdraht aus der Elektrode herausgezogen und durch einen steiferen Mandrin ersetzt. Mit diesem Mandrin ist das Anstemmen der Elektrode möglich. Ein derartiger Mandrin kann auch genutzt werden, um die Elektrode zu Beginn der Operation auszurichten und in den Körper einzuführen.

Als problematisch erweist sich, dass die Steifigkeit des Mandrins die Führung der Elektrode in die Zielvene stark behindert. Nach dem Einbringen der Elektrode in den Körper wird deshalb in der Regel auf einen Führungsdraht gewechselt. Ferner problematisch erweist sich, dass aufgrund der geringen Steifigkeit des Führungsdrahtes in der Zielvene ein Anstemmen der Elektrode über den Führungsdraht kaum möglich ist. Somit ist ein erneuter Wechsel zurück zum Mandrin erforderlich. Dabei kommt es jedoch häufig zu einem Verrutschen der Elektrode. Ferner kann ein Verrutschen der Elektrode auftreten, wenn der Mandrin zum Anstemmen in die Elektrode hinein geführt wird. Als Konsequenz kann eine iterativ wechselnde Nutzung von Führungsdraht und Mandrin erforderlich sein. Durch diese Schwierigkeiten werden die Dauer der Operation und damit auch die Kosten und das Risiko für den Patienten erhöht.

Ein oder mehrere der geschilderten Nachteile des Standes der Technik werden mit Hilfe der Erfindung behoben oder zumindest gemindert. Ein erster Aspekt der Erfindung betrifft dazu ein Elektrodenimplantationssystem, umfassend:
A) wenigstens ein längliches Implantationswerkzeug, das einen proximalen Abschnitt, der wenigstens abschnittsweise als Mandrin ausgebildet ist, und einen distalen Abschnitt, der als flexibler Führungsdraht ausgebildet ist, umfasst, wobei der proximale und der distale Abschnitt durch ein statisches oder verstellbares Anschlagelement voneinander abgegrenzt ist und das Anschlagelement eine dem distalen Abschnitt zugewandte Anschlagoberfläche umfasst; und
B) eine implantierbare Elektrode mit einer Elektrodenhülse, die einen zu der Anschlagoberfläche komplementären Anschlagbereich aufweist, der dem proximalen Abschnitt zugewandt ist.
Die Elektrodenhülse und das längliche Implantationswerkzeug sind wenigstens abschnittsweise koaxial miteinander angeordnet und entlang sowie um eine gemeinsamen Längsachse relativ zueinander verlagerbar.

Das Elektrodenimplantationssystem bietet den Vorteil, dass eine Implantation der Elektrode möglich ist, ohne dass dabei zwischen unterschiedlichen Werkzeugen hin- und hergewechselt werden muss. Das Elektrodenimplantationssystem ermöglicht somit eine einfache, sichere und schnelle Implantation der Elektrode. So kann das längliche Implantationswerkzeug zunächst in die Elektrodenhülse eingeführt beziehungsweise durch diese hindurch geführt werden. Der als Führungsdraht ausgebildete distale Abschnitt des länglichen Implantationswerkzeugs kann dann in den Körper des Patienten eingeführt werden und zur Navigation durch die Gefäße verwendet werden. Das längliche Implantationswerkzeug kann so lange durch die Elektrodenhülse hindurchgeschoben werden, bis die Anschlagoberfläche des Anschlagelements an dem komplementären Anschlagbereich der Elektrodenhülse zum Anliegen kommt. Somit wird die Elektrode, die sich in diesem Zustand abschnittsweise auf dem als Mandrin ausgebildeten proximalen Abschnitt befindet, ausgerichtet und kann auf einfache Weise in den Körper des Patienten eingeführt werden.

In weiteren Ausführungsformen kann die Elektrode auch über das Anschlagelement hinaus, vollständig auf den proximalen Abschnitt geschoben werden. Solche Ausführungsformen des Elektrodenimplantationssystems werden im Folgenden noch beschrieben. Anschließend kann die Elektrode auf dem länglichen Implantationswerkzeug wieder in Richtung des distalen Abschnitts verlagert werden und über diesen in die Zielvene navigiert werden. In der Zielvene kann das längliche Implantationswerkzeug mit der Anschlagoberfläche dann wieder an dem komplementären Anschlagbereich der Elektrodenhülse zum Anliegen gebracht werden und eine Anstemmkraft auf die Elektrodenhülse übertragen werden. Nachdem die Elektrode sicher in dem Gefäß befestigt worden ist, kann das längliche Implantationswerkzeug zurückgezogen und aus der Elektrode und letztendlich aus dem Körper des Patienten entfernt werden.

Der Begriff 'proximal' wird im Zusammenhang mit der vorliegenden Erfindung definiert, als in Richtung der Körpermitte eines Verwenders des Elektrodenimplantationssystems weisend. Der Begriff Verwender bezeichnet dabei eine handhabende Person, also in der Regel einen Arzt beziehungsweise Operateur. Dementsprechend wird der Begriff "distal" definiert, als entgegen der Richtung der Körpermitte des Verwenders des Elektrodenimplantationssystems weisend. Mit anderen Worten, der Begriff distal bezeichnet eine Richtung hin zum Patienten. Diese Definitionen gelten analog, wenn von dem länglichen Implantationswerkzeug oder der Elektrode beziehungsweise Elektrodenhülse allein gesprochen wird.

Der Begriff der "gemeinsamen Längsachse" bezieht sich auf jenen Bereich, in welchem das längliche Implantationswerkzeug konzentrisch in der Elektrodenhülse angeordnet ist. Die Elektrodenhülse weist formstabile Eigenschaften auf Somit passt sich das längliche Implantationswerkzeug, welches zumindest anteilig elastische Eigenschaften oder biegeschlaffe Eigenschaften aufweist, an die Form einer Durchgangsbohrung der Elektrodenhülse an. In diesem Bereich sind eine Längsachse der Elektrodenhülse und eine Längsachse des länglichen Implantationswerkzeugs kollinear zueinander ausgerichtet und somit identisch. Dem Fachmann ist ohne weiteres bewusst, dass das längliche Implantationswerkzeug, ähnlich einem Schlauch, einem dünnen Röhrchen oder einem dünnen Draht, unabhängig von der Elektrodenhülse Biegelinien annehmen kann. Seine Längsachse kann dann auch eine geschwungene Linie darstellen. Wenn im Folgenden allein von der Längsachse des länglichen Implantationswerkzeugs gesprochen wird, so bezieht sich dies auf einen Zustand, in dem das längliche Implantationswerkzeug vollständig oder in einem betreffenden Abschnitt linear ausgestreckt vorliegt.

In einer ersten Variante einer bevorzugten Ausführungsform des erfindungsgemäßen Elektrodenimplantationssystems ist vorgesehen, dass das Anschlagelement einen um die gemeinsame Längsachse rotationssymmetrischen Bereich umfasst, der quer zur gemeinsamen Längsachse gegenüber dem distalen Abschnitt einen größeren Querschnitt aufweist und in jeder relativen Orientierungen des länglichen Implantationswerkzeugs und der Elektrodenhülse um die gemeinsame Längsachse eine Projektion des komplementären Anschlagbereichs entlang der gemeinsamen Längsachse mit der Anschlagoberfläche überlappt.

Mit anderen Worten ausgedrückt, weist das längliche Implantationswerkzeug gemäß der vorangehenden Ausführungsform einen um seine Längsachse ausgetragenen Rotationskörper auf, der zu einer Querschnittserhöhung führt. Bevorzugt sind der proximale Abschnitt und der distale Abschnitt ebenfalls als Rotationskörper ausgebildet. Der distale Abschnitt kann bevorzugt eine Länge von 10 mm bis 300 mm aufweisen, insbesondere 20 mm bis 200 mm. Ein Querschnittsdurchmesser des Anschlagelements ist stets größer, als ein Querschnittsdurchmesser des distalen Abschnitts. Der Querschnittsdurchmesser des Anschlagelements kann größer oder gleich dem des proximalen Abschnitts sein. Wird die Elektrodenhülse mit dem komplementären Anschlagbereich lateral entlang der gemeinsamen Längsachse in Richtung des Anschlagelements verlagert, so kommt es mit seinem komplementären Anschlagbereich an diesem zum Anliegen. Es ist in dieser Ausführungsform dabei unabhängig, wie die Elektrodenhülse und das längliche Implantationswerkzeug relativ zueinander um die gemeinsame Längsachse verdreht sind. Durch das Anschlagelement wird also die laterale Beweglichkeit der Elektrode auf dem länglichen Implantationswerkzeug einseitig beschränkt. Das Elektrodenimplantationssystem der Erfindung bietet den Vorteil, dass es besonders einfach aufgebaut ist und damit günstig herstellbar und einfach handhabbar ist.

In weiter bevorzugter Ausgestaltung der zuvor beschriebenen ersten Variante des Elektrodenimplantationssystems ist vorgesehen, dass das Anschlagelement als kugelförmige oder ringförmige Verdickung des länglichen Implantationswerkzeugs ausgebildet ist. Dies bietet den Vorteil, dass sprunghafte Querschnittsänderungen und beispielsweise scharfe Kanten an dem Anschlagelement vermieden werden. Somit lässt sich das Anschlagelement besonders einfach in die Elektrodenhülse bis zu dem komplementären Anschlagbereich einführen.

Gemäß einer zweiten Variante des Elektrodenimplantationssystems ist vorgesehen, dass das Anschlagelement einen um die gemeinsame Längsachse rotations-asymmetrischen Bereich umfasst, der quer zur gemeinsamen Längsachse gegenüber dem distalen Abschnitt einen erhöhten Querschnitt aufweist und eine begrenzte Anzahl an relativen Orientierungen des länglichen Implantationswerkzeugs und der Elektrodenhülse um die gemeinsame Längsachse existiert, in denen eine Projektion des komplementären Anschlagbereichs entlang der gemeinsamen Längsachse an keiner Stelle mit der Anschlagoberfläche überlappt.

Der Begriff des 'rotations-asymmetrischen Bereichs' lässt sich veranschaulichen, in dem man sich zunächst einen rotations-symmetrischen Bereich vorstellt, von dem dann entlang der Längsachse des länglichen Implantationswerkzeugs abschnittsweise Material entfernt wird. Das Material wird bezogen auf die Längsachse des länglichen Implantationswerkzeugs radial von außen nach innen entfernt. Somit ergeben sich in radialer Richtung unterschiedliche Querschnittsdurchmesser des rotations-symmetrischen Bereichs. Der komplementäre Anschlagbereich der Elektrodenhülse ist nun derart gestaltet, dass der rotations-asymmetrische Bereich des Anschlagelements, in beispielsweise einer einzigen rotatorischen relativen Orientierung des länglichen Implantationswerkzeugs und der Elektrodenhülse um die gemeinsame Längsachse, lateral entlang der gemeinsamen Längsachse durch den komplementären Anschlagbereich der Elektrodenhülse hindurchschiebbar ist. In allen anderen rotatorischen relativen Orientierungen kommt die Anschlagoberfläche des Anschlagelements hingegen an dem komplementären Anschlagbereich zum Anliegen. Dies bietet den Vorteil, dass durch Veränderung der relativen rotatorischen Orientierung des länglichen Implantationswerkzeugs und der Elektrode zwischen zwei Zuständen gewechselt werden kann. In einem ersten Zustand sind die Elektrode und das längliche Implantationswerkzeug über die gesamte Länge des länglichen Implantationswerkzeugs entlang der gemeinsamen Längsachse verschiebbar zueinander. In einem zweiten Zustand ist die Verschiebbarkeit durch das Anschlagelement und den komplementären Anschlagbereich von distal nach proximal begrenzt. Die Flexibilität der Handhabung des Elektrodenimplantationssystems wird dadurch signifikant erhöht. Beispielsweise kann das längliche Implantationswerkzeugs aus zwei wählbaren Richtungen in die Elektrodenhülse eingeführt werden. Das Anschlagelement und der komplementäre Anschlagbereich können in dieser Ausführungsform hinsichtlich ihrer Funktionalität mit einem Schlüssel-Schloss-Prinzip beschrieben werden. Der distale Abschnitt kann bevorzugt eine Länge von 10 mm bis 60 mm aufweisen, insbesondere 20 mm bis 50 mm. Der proximale Abschnitt kann hinsichtlich der Größe seines Querschnitts auch aus proximaler Richtung in distaler Richtung bis hin zu dem Anschlagelement kontinuierlich oder als Profil ansteigen. Somit wird eine Zentrierung der Elektrodenhülse auf dem länglichen Implantationswerkzeug in Richtung des Anschlagelements unterstützt. Dies vereinfacht das Schlüssel-Schloss-Prinzip signifikant.

In weiter bevorzugter Ausgestaltung der zweiten Variante ist vorgesehen, dass das Anschlagelement als Verdickung des länglichen Implantationswerkzeugs ausgebildet ist, die im Bereich der Anschlagoberfläche eine quer zur gemeinsamen Längsachse polygonale oder elliptische Querschnittsform aufweist. Dies bietet den Vorteil, dass die Querschnittsformen mit geringem Aufwand herstellbar und im Sinne des Schlüssel-Schloss-Prinzips einfach handhabbar sind.

In einer weiteren, dritten Variante des erfindungsgemäßen Elektrodenimplantationssystems ist vorgesehen, dass das längliche Implantationswerkzeug zweiteilig ausgeführt ist und einen als flexiblen Führungsdraht ausgebildeten inneren Teil sowie einen als Mandrin ausgebildeten äußeren Teil umfasst und der äußere Teil mit einer inneren Mantelfläche auf einer äußeren Mantelfläche des inneren Teils verlagerbar angeordnet ist, so dass der distale Abschnitt des länglichen Implantationswerkzeugs von dem inneren Teil gebildet wird und der proximale Abschnitt von dem inneren Teil und dem äußeren Teil gebildet wird, wobei der äußere Teil das Anschlagelement umfasst.

In dieser Ausführungsform sind der proximale Abschnitt und der distale Abschnitt somit flexibel hinsichtlich ihrer jeweiligen Länge anpassbar. Der äußere Teil, der als Mandrin ausgebildet ist, ist auf den inneren Teil, der als Führungsdraht ausgebildet ist, aufgeschoben. Je nachdem, wie weit der äußere Teil auf den inneren Teil aufgeschoben wird, lassen sich die jeweiligen Längen des distalen Abschnitts, der als Führungsdraht ausgebildet, und des proximalen Abschnitts, der als Führungsdraht und als diesen ummantelnder Mandrin ausgebildet ist, flexibel einstellen. Der innere Teil kann beispielsweise als konventioneller Führungsdraht mit beispielsweise einem Durchmesser von 0,36 mm ausgebildet sein. Der äußere Teil kann beispielsweise als flexibles hohlzylinderförmiges Element mit beispielsweise einem Innendurchmesser von 0,38 mm und einem Außendurchmesser von 0,48 mm ausgebildet sein. Der innere und der äußere Teil können aus gleichen oder unterschiedlichen Materialien hergestellt sein.

Bevorzugt befindet sich bei der vorgenannten Variante die Anschlagoberfläche des Anschlagelements an einem distalen Endbereich des äußeren Teils. Diese Ausführungsform bietet den Vorteil, dass die Länge des distalen Abschnitts und damit der verfügbaren Länge des Führungsdrahts flexibel anpassbar ist. Dies ist insbesondere in stark verzweigten und abgewinkelten Gefäßen vorteilhaft für die Navigation der Elektrode. Ein weiterer Vorteil dieser Ausführungsform besteht darin, dass der innere Teil als Führungsdraht vollständig aus dem äußeren Teil herausgezogen werden kann, wenn die Elektrode ihren Zielort erreicht hat und die Elektrode dabei mit dem äußeren Teil angestemmt werden kann. Somit ist das Elektrodenimplantationssystem besonders einfach handhabbar und besonders flexibel. Da die einzelnen Elemente des länglichen Implantationswerkzeugs separat voneinander herstellbar sind, ergeben sich zu dem Vorteile hinsichtlich des Produktionsaufwands und der Qualität, beispielsweise im Falle einer Produktion in größeren Serien.

In weiter bevorzugter Ausgestaltung der dritten Variante des erfindungsgemäßen Elektrodenimplantationssystems ist vorgesehen, dass der äußere Teil eine schlauchartige oder wendelartige Geometrie aufweist und die Anschlagsoberfläche an einem distalen Endbereich der schlauchartigen oder wendelartigen Geometrie vorgesehen ist. Solche Geometrien sind vorteilhaft mit geringem Aufwand herstellbar und eignen sich für eine einfache und flexible Handhabung. Durch entsprechende Gestaltung der schlauchartigen oder wendelartigen Geometrie lassen sich unterschiedlichste mechanische Eigenschaften flexibel herstellen. Beispielsweise kann die schlauchartige Geometrie geschlossen, also hohlzylinderförmig sein. Es sind aber auch hohlzylinderförmige Geometrien mit beispielsweise in der Mantelfläche eingebrachten langlochförmigen Schlitzen möglich, die sich entlang der Längsachse der schlauchartigen Geometrie erstrecken. Weiterhin möglich sind in der Mantelfläche eingebrachte langlochförmige Schlitze, die sich auf jeweils einem Umfang oder Teilumfang um die Mantelfläche herum erstrecken. Hinsichtlich der wendelartigen Geometrie lässt sich beispielsweise eine Wendesteigung variieren. Diese kann zum Beispiel konstant sein oder über die Längserstreckung der wendelförmigen Geometrie ein Steigungsprofil aufweisen. Somit lässt sich insbesondere ein Steifigkeitsprofil des äußeren Teils flexibel an die Anforderungen der Operation und des Patienten anpassen.

Grundsätzlich lässt sich für alle Ausführungsformen des Elektrodenimplantationssystems der Erfindung sagen, dass ein Steifigkeitsprofil des Mandrins, welches in proximaler Richtung eine höhere Steifigkeit aufweist und in distaler Richtung eine geringere Steifigkeit aufweist, vorteilhaft ist. Je weiter der Operateur mit dem Elektrodenimplantationssystem in den Körper des Patienten vordringt, umso besser bleibt somit die Möglichkeit der gezielten Navigation mit dem distalen Abschnitt erhalten. Andere Steifigkeitseigenschaften des Mandrins und des Führungsdrahtes sowie des länglichen Implantationswerkzeugs insgesamt wählt der zuständige Fachmann selbstständig aus. Dabei berücksichtigt er insbesondere die vorliegenden Anforderungen des Operationsprozesses und des Patienten. Als Werkstoffe für das längliche Implantationswerkzeug kommen alle Werkstoffe in Betracht, die für Führungsdrähte und Mandrine bekanntermaßen geeignet sind.

Ein weiterer Aspekt der vorliegenden Erfindung betrifft ein längliches Implantationswerkzeug, umfassend:
- einen proximalen Abschnitt, der wenigstens abschnittsweise als Mandrin ausgebildet ist;
- einen distalen Abschnitt, der als flexibler Führungsdraht ausgebildet ist; und
- ein statisches oder verstellbares Anschlagelement, das den proximalen und den distalen Abschnitt voneinander abgegrenzt und das eine dem distalen Abschnitt zugewandte Anschlagoberfläche umfasst.

In einer bevorzugten Ausführungsform des länglichen Implantationswerkzeugs ist vorgesehen, dass das Anschlagelement einen um eine Längsachse des länglichen Implantationswerkzeugs rotationssymmetrischen Bereich umfasst, der quer zu der Längsachse gegenüber dem distalen Abschnitt einen größeren Querschnitt aufweist.

In einer weiteren bevorzugten Ausführungsform des länglichen Implantationswerkzeugs ist vorgesehen, dass das Anschlagelement einen um eine Längsachse rotations-asymmetrischen Bereich umfasst, der quer zu der Längsachse gegenüber dem distalen Abschnitt einen erhöhten Querschnitt aufweist. Insbesondere ist das Anschlagelement als Verdickung des länglichen Implantationswerkzeugs ausgebildet, die im Bereich der Anschlagoberfläche eine quer zu der Längsachse polygonale oder elliptische Querschnittsform aufweist.

In einer weiteren bevorzugten Ausführungsform des länglichen Implantationswerkzeugs ist vorgesehen, dass es zweiteilig ausgeführt ist und einen als flexiblen Führungsdraht ausgebildeten inneren Teil sowie einen als Mandrin ausgebildeten äußeren Teil umfasst und der äußere Teil mit einer inneren Mantelfläche auf einer äußeren Mantelfläche des inneren Teils verlagerbar angeordnet ist, so dass der distale Abschnitt von dem inneren Teil gebildet wird und der proximale Abschnitt von dem inneren Teil und dem äußeren Teil gebildet wird, wobei der äußere Teil das Anschlagelement umfasst. Insbesondere weist der äußere Teil eine schlauchartige oder wendelartige Geometrie auf und die Anschlagsoberfläche ist an einem distalen Endbereich der schlauchartigen oder wendelartigen Geometrie vorgesehen.

Ein weiterer Aspekt der vorliegenden Erfindung betrifft eine implantierbare Elektrode, welche eine Elektrodenhülse umfasst, die einen Anschlagbereich aufweist, der sich quer zu einer Längsachse der Elektrodenhülse erstreckt. In einer bevorzugten Ausführungsform der implantierbaren Elektrode ist vorgesehen, dass der Anschlagbereich einen polygonalen oder elliptischen Ausschnitt aufweist.

Weitere bevorzugte Ausgestaltungen das erfindungsgemäßen Elektrodenimplantationssystems, des erfindungsgemäßen länglichen Implantationswerkzeugs und der erfindungsgemäßen implantierbaren Elektrode ergeben sich aus der nachfolgenden Beschreibung.

Offenbart wird zudem eine technische Lehre zur Durchführung eines Verfahrens zur Implantation einer Elektrode.

Die vorliegende Erfindung wird nachfolgend anhand einiger Ausführungsbeispiele näher erläutert. Es zeigen:
- Fig. 1 a/b: eine prinziphafte Darstellung des erfindungsgemäßen Elektrodenimplantationssystems gemäß einer ersten Variante;
- Fig. 2 a-c: eine prinziphafte Darstellung des erfindungsgemäßen Elektrodenimplantationssystems gemäß einer zweiten Variante; und
- Fig. 3 a-g: eine prinziphafte Darstellung des erfindungsgemäßen Elektrodenimplantationssystems gemäß einer dritten Variante.

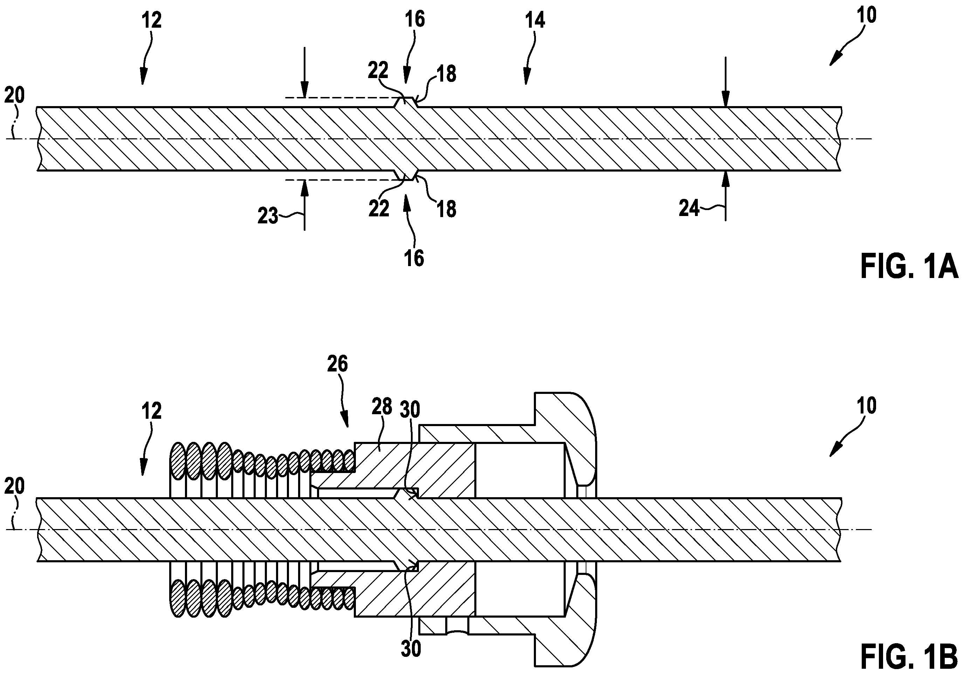

Den Figuren 1a und 1b sind zwei Schnittansichten durch erfindungswesentliche Bereiche des Elektrodenimplantationssystems gemäß einer ersten Variante zu entnehmen. Figur 1a zeigt dabei ein längliches Implantationswerkzeug 10, das einen proximalen Abschnitt 12 und einen distalen Abschnitt 14 umfasst, die in diesem Ausführungsbeispiel durch ein statisches Anschlagelement 16 voneinander abgegrenzt sind. Der proximale Abschnitt 12 ist als Mandrin ausgebildet und weist höhere Steifigkeitseigenschaften auf, als der distale Abschnitt 14, der als flexibler Führungsdraht ausgebildet ist.

Das Anschlagelement 16 weist eine Anschlagoberfläche 18 auf, die dem distalen Abschnitt 14 zugewandt ist. In diesem Ausführungsbeispiel ist das Anschlagelement 16 als ringförmige Verdickung des länglichen Implantationswerkzeugs 10 ausgebildet. Das Anschlagelement 16 umfasst also einen um seine Längsachse 20 rotationssymmetrisch ausgetragenen Bereich 22, der quer zu einer Längsachse 20 einen Querschnitt 23 aufweist, der gegenüber einem Querschnitt 24 des distalen Abschnitts 14 erhöht ist.

Figur 1b zeigt das längliche Implantationswerkzeug 10 mit einer abschnittsweise dargestellten implantierbaren Elektrode 26. Die implantierbare Elektrode 26 umfasst eine Elektrodenhülse 28. Die Elektrodenhülse 28 und das längliche Implantationswerkzeugs 10 sind koaxial miteinander angeordnet. Dies bedeutet, die Elektrodenhülse 28 und das längliche Implantationswerkzeugs 10 weisen wenigstens über die Länge der Elektrodenhülse 28 eine gemeinsame Längsachse 20 auf. Transversal entlang und rotatorischen um diese gemeinsamen Längsachse 20 sind die Elektrodenhülse 28 und das Implantationswerkzeugs 10 relativ zueinander verlagerbar.

Die Elektrodenhülse 28 weist einen zu der Anschlagoberfläche 18 komplementären Anschlagbereich 30 auf. Der Anschlagbereich 30 ist dem proximalen Abschnitt 12 zugewandt. In jeder möglichen relativen Orientierung des länglichen Implantationswerkzeugs 10 und der Elektrodenhülse 28 um die gemeinsame Längsachse 20 überlappt eine Projektion des komplementären Anschlagbereichs 30 in Richtung der Anschlagoberfläche 18 und entlang der gemeinsamen Längsachse 20 mit der Anschlagoberfläche 18. Mit anderen Worten ausgedrückt, wird eine Verschiebbarkeit der Elektrode 26 auf dem länglichen Implantationswerkzeugs 10 in Richtung des proximalen Abschnitts 12 dadurch begrenzt, dass eine Wirkflächenpaarung zwischen der Anschlagoberfläche 18 und dem komplementären Anschlagbereich 30 auftritt.

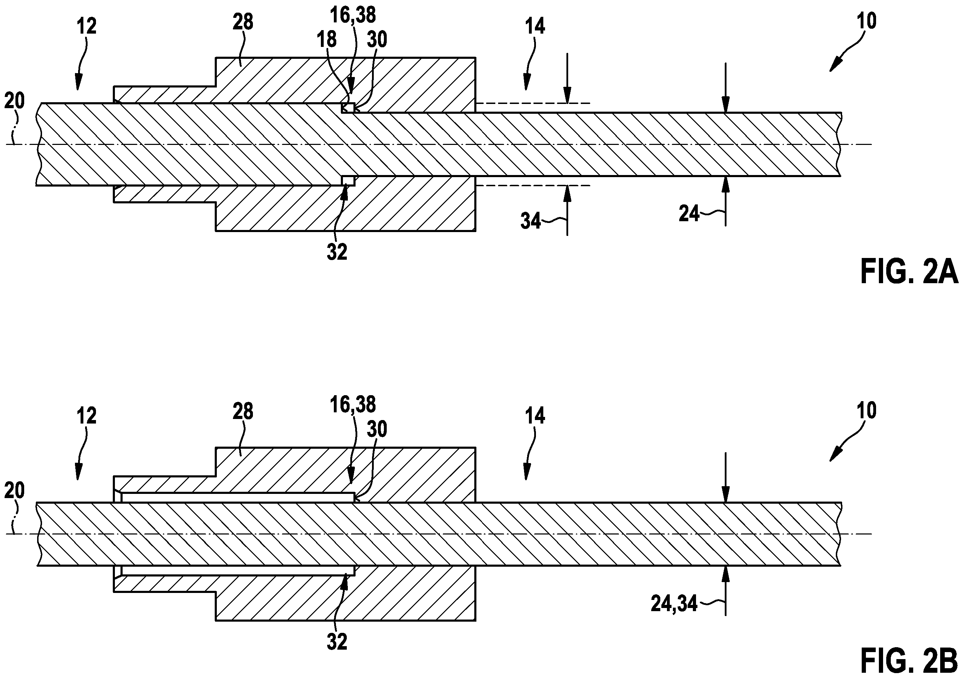

Den Figuren 2a und 2b sind zwei zueinander um 90° versetzte Querschnittansichten durch denselben erfindungswesentlichen Bereich des Elektrodenimplantationssystems gemäß einer zweiten Variante zu entnehmen. Das zu den Figur 1a und 1b Beschriebene gilt im Wesentlichen auch für die Figuren 2a und 2b, sodass hier lediglich auf die Unterschiede eingegangen wird und teilweise identische Bezugszeichen verwendet werden.

Figur 2a zeigt das erfindungsgemäße Elektrodenimplantationssystem in einer Querschnittsansicht und zwar in dem Bereich, in dem sich die Elektrodenhülse 28 auf dem länglichen Implantationswerkzeugs 10 befindet. Die implantierbare Elektrode 26 insgesamt ist hier nicht weiter dargestellt. In diesem Ausführungsbeispiel umfasst das Anschlagelement 16 einen um die gemeinsame Längsachse 20 rotations-asymmetrischen Bereich 32. Dieser weist quer zu der gemeinsamen Längsachse 20 einen Querschnitt 34 auf, der gegenüber dem Querschnitt 24 des distalen Abschnitts 14 erhöht ist. Durch Verlagerung der Elektrodenhülse 28 entlang der gemeinsamen Längsachse 20 in Richtung des proximalen Abschnitts 12 lässt sich der komplementäre Anschlagbereich 30 der Elektrodenhülse 28 mit der Anschlagoberfläche 18 des Anschlagelements 16 des länglichen Implantationswerkzeugs 10 in Kontakt bringen.

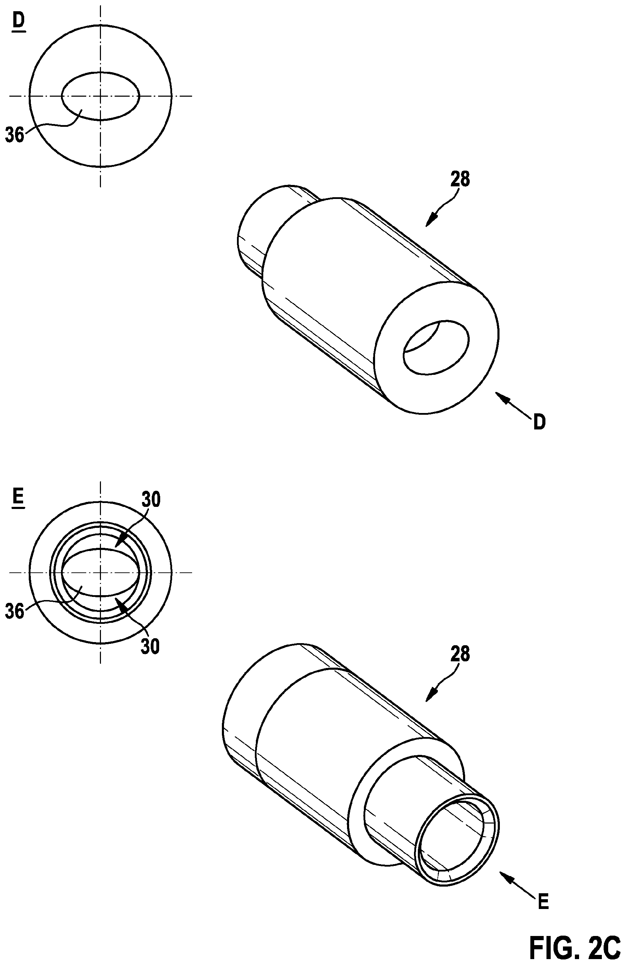

Verglichen mit Figur 2a sind in Figur 2b das längliche Implantationswerkzeugs 10 und die Elektrodenhülse 28 relativ zueinander um 90° um die gemeinsame Längsachse 20 verdreht. Aus Figur 2b ist daher ersichtlich, dass das Anschlagelement 16 in dem rotations-asymmetrischen Bereich 32 nun einen Querschnitt 34 aufweist, der gegenüber dem Querschnitt 24 des distalen Abschnitts 14 nicht erhöht ist. Der rotations-asymmetrischen Bereich 32 des Anschlagelements 16 und der komplementäre Anschlagbereich 30 bilden somit einer Art Schlüssel-Schloss-System, in welchem sich die Anschlagoberfläche 18 des Anschlagelements 16 mit einem entsprechenden Ausschnitt 36 (vergleiche Figur 2c) in dem komplementären Anschlagbereich 30 kongruent übereinanderlegen lassen. Somit kann die Elektrodenhülse 28 in dieser Orientierung auch über das Anschlagelement 16 hinaus vollständig auf den proximalen Abschnitt 12 geschoben werden. Je nach konstruktiver Ausgestaltung des rotations-asymmetrischen Bereichs 32 und des komplementären Anschlagbereichs 30 existiert eine begrenzte Anzahl an relativen Orientierungen des Implantationswerkzeugs 10 und der Elektrodenhülse 28 um die gemeinsame Längsachse 20, in denen dies möglich ist. Anders ausgedrückt existiert dann eine begrenzte Anzahl an relativen Orientierungen, in denen eine Projektion das komplementären Anschlagbereichs 30 entlang der gemeinsamen Längsachse 20 und in Richtung des Anschlagelements 16 an keiner Stelle mit der Anschlagoberfläche 18 überlappt. Aus Figur 2a geht hervor, dass das längliche Implantationswerkzeugs 10 sich im proximalen Abschnitt 12 in Richtung des distalen Abschnitts 14 aufweitet und sich in umgekehrter Richtung verjüngt.

Figur 2c zeigt die Elektrodenhülse 28 isoliert von den anderen Elementen des erfindungsgemäßen Elektrodenimplantationssystems. Die Ansichten D und E der Elektrodenhülse 28 zeigen, dass der komplementäre Anschlagbereich 30 den Ausschnitt 36 umfasst, der in diesem Ausführungsbeispiel eine elliptische Querschnittsform aufweist. Das in Figur 2c nicht dargestellte Anschlagelement 16 aus den Figuren 2a und 2b, das eine Verdickung 38 des Implantationswerkzeugs 10 darstellt, kann im Bereich der Anschlagsoberfläche 18 dann ebenfalls eine quer zu der gemeinsamen Längsachse 20 elliptische Querschnittsform aufweisen. Somit passen das Anschlagelement 16 und der komplementäre Anschlagbereich 30 im Sinne des Schlüssel-Schloss-Prinzips zusammen.

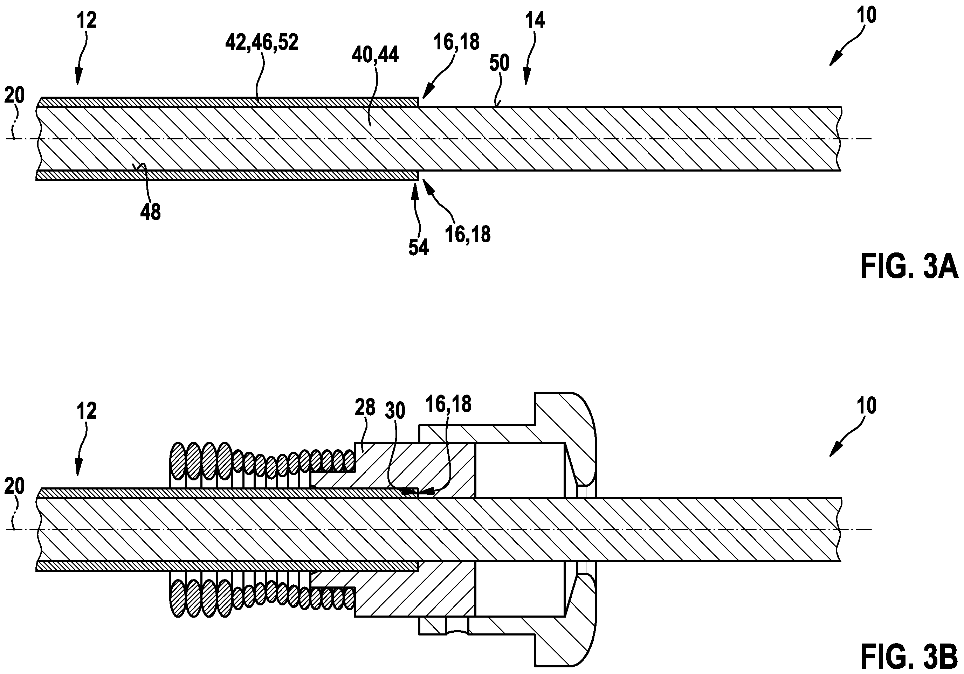

Den Figuren 3a und 3b sind zwei Schnittansichten durch erfindungswesentliche Bereiche des Elektrodenimplantationssystems gemäß einer dritten Variante zu entnehmen. Sofern das zu vorangehenden Varianten Beschriebene auch für die Ausführungsform gemäß der Figuren 3a und 3b gilt, werden identische Bezugszeichen verwendet.

In den Figuren 3a und 3b ist eine Ausführungsform des erfindungsgemäßen Elektrodenimplantationssystems gezeigt, in welcher das längliche Implantationswerkzeugs 10 zweiteilig ausgeführt ist. Figur 3a zeigt eine Querschnittsansicht des länglichen Implantationswerkzeugs 10. Dieses umfasst einen inneren Teil 40 und einen äußeren Teil 42. Der innere Teil 40 ist als flexibler Führungsdraht 44 und der äußere Teil 42 ist als Mandrin 46 ausgebildet. Der äußere Teil 42 umfasst eine innere Mantelfläche 48 und der innere Teil 40 umfasst eine äußere Mantelfläche 50. Der äußere Teil 42 ist mit seiner inneren Mantelfläche 48 über die äußere Mantelfläche 50 des inneren Teils 40 geschoben und auf dieser verlagerbar. Der äußere Teil 42 weist in diesem Ausführungsbeispiel eine schlauchartige Geometrie 52 auf. Der äußere Teil 42 umfasst hier auch das Anschlagelement 16. Die Anschlagoberfläche 18 ist dabei an einem distalen Endbereich 54 der schlauchartigen Geometrie 52 vorgesehen. Das Anschlagelement 16 ist somit hinsichtlich seiner Lage auf dem inneren Teil 40 verstellbar. Da das Anschlagelement 16 den proximalen Abschnitt 12 und den distalen Abschnitt 14 voneinander abgegrenzt, ist die jeweilige Länge des proximalen Abschnitts 12 und des distalen Abschnitts 14 flexibel einstellbar. Der distale Abschnitt 14 wird dabei allein von dem inneren Teil 40 gebildet. Der proximale Abschnitt 12 hingegen wird von dem inneren Teil 40 und dem äußeren Teil 42 gemeinsam gebildet.

Figur 3b zeigt das längliche Implantationswerkzeugs 10 und die implantierbare Elektrode 26 mit der Elektrodenhülse 28 in einer Querschnittsansicht. Es ist ersichtlich, dass die Anschlagoberfläche 18 des Anschlagelements 16 in dem gezeigten Zustand an dem komplementären Anschlagbereich 30 der Elektrodenhülse 28 anliegt.

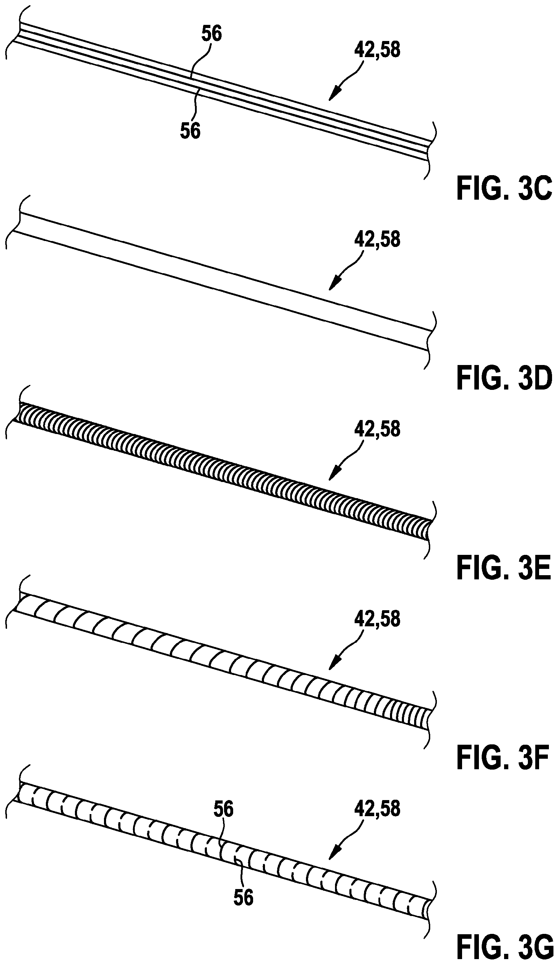

Die Figuren 3c bis 3g zeigen unterschiedliche Ausführungsformen des äußeren Teils 42. Figur 3c zeigt ein Ausführungsbeispiel des äußeren Teils 42, das eine hohlzylinderförmige oder auch schlauchartige Geometrie 52 aufweist und das zudem langlochförmige Schlitze 56 aufweist, die in die Mantelfläche des äußeren Teils 42 eingebracht sind und die sich entlang dessen Längsachse erstrecken. Gemäß Figur 3g können sich die langlochförmigen Schlitze 56 auch auf jeweils einem Umfang oder Teilumfang um die Mantelfläche herum erstrecken. Figur 3d zeigt ein Ausführungsbeispiel des äußeren Teils 42, das in rein schlauchartiger Geometrie 52 ausgeführt ist. In Figur 3e ist eine wendelartige Geometrie 58 gezeigt, mit konstanter Wendelsteigung. Figur 3f zeigt eine wendelartige Geometrie 58, die eine in Richtung des hier nicht dargestellten distalen Abschnitts 14 fallende Wendelsteigung aufweist.

## Patentansprüche

1. Elektrodenimplantationssystem, umfassend:
A) wenigstens ein längliches Implantationswerkzeug (10), das einen proximalen Abschnitt (12), der wenigstens abschnittsweise als Mandrin ausgebildet ist, und einen distalen Abschnitt (14), der als flexibler Führungsdraht ausgebildet ist, umfasst, wobei der proximale und der distale Abschnitt (12, 14) durch ein statisches oder verstellbares Anschlagelement (16) voneinander abgegrenzt sind und das Anschlagelement (16) eine dem distalen Abschnitt (14) zugewandte Anschlagoberfläche (18) umfasst; und
B) eine implantierbare Elektrode (26) mit einer Elektrodenhülse (28), die einen zu der Anschlagoberfläche (18) komplementären Anschlagbereich (30) aufweist, der dem proximalen Abschnitt (12) zugewandt ist,
wobei die Elektrodenhülse (28) und das längliche Implantationswerkzeug (10) wenigstens abschnittsweise koaxial miteinander angeordnet und entlang sowie um eine gemeinsamen Längsachse (20) relativ zueinander verlagerbar sind.

2. Elektrodenimplantationssystem nach Anspruch 1, **dadurch gekennzeichnet, dass** das Anschlagelement (16) einen um die gemeinsame Längsachse (20) rotationssymmetrischen Bereich (22) umfasst, der quer zur gemeinsamen Längsachse (20) gegenüber dem distalen Abschnitt (14) einen größeren Querschnitt (23) aufweist und in jeder relativen Orientierungen des länglichen Implantationswerkzeugs (10) und der Elektrodenhülse um die gemeinsame Längsachse (20) eine Projektion des komplementären Anschlagbereichs (30) entlang der gemeinsamen Längsachse (20) mit der Anschlagoberfläche (18) überlappt.

3. Elektrodenimplantationssystem nach Anspruch 2, **dadurch gekennzeichnet, dass** das Anschlagelement (16) als kugelförmige oder ringförmige Verdickung des länglichen Implantationswerkzeugs (10) ausgebildet ist.

4. Elektrodenimplantationssystem nach Anspruch 1, **dadurch gekennzeichnet, dass** das Anschlagelement (16) einen um die gemeinsame Längsachse (20) rotations-asymmetrischen Bereich (32) umfasst, der quer zur gemeinsamen Längsachse (20) gegenüber dem distalen Abschnitt (14) einen erhöhten Querschnitt (34) aufweist und eine begrenzte Anzahl an relativen Orientierungen des länglichen Implantationswerkzeugs (10) und der Elektrodenhülse (28) um die gemeinsame Längsachse (20) existiert, in denen eine Projektion des komplementären Anschlagbereichs (30) entlang der gemeinsamen Längsachse (20) an keiner Stelle mit der Anschlagoberfläche (18) überlappt.

5. Elektrodenimplantationssystem nach Anspruch 4, **dadurch gekennzeichnet, dass** das Anschlagelement (16) als Verdickung (38) des länglichen Implantationswerkzeugs (10) ausgebildet ist, die im Bereich der Anschlagoberfläche (18) eine quer zur gemeinsamen Längsachse (20) polygonale oder elliptische Querschnittsform aufweist.

6. Elektrodenimplantationssystem nach Anspruch 1 oder 2, **dadurch gekennzeichnet, dass** das längliche Implantationswerkzeug (10) zweiteilig ausgeführt ist und einen als flexiblen Führungsdraht (44) ausgebildeten inneren Teil (40) sowie einen als Mandrin (46) ausgebildeten äußeren Teil (42) umfasst und der äußere Teil (42) mit einer inneren Mantelfläche (48) auf einer äußeren Mantelfläche (50) des inneren Teils (40) verlagerbar angeordnet ist, so dass der distale Abschnitt (14) des länglichen Implantationswerkzeugs (10) von dem inneren Teil (40) gebildet wird und der proximale Abschnitt (12) von dem inneren Teil (40) und dem äußeren Teil (42) gebildet wird, wobei der äußere Teil (42) das Anschlagelement (16) umfasst.

7. Elektrodenimplantationssystem nach Anspruch 6, **dadurch gekennzeichnet, dass** der äußere Teil (42) eine schlauchartige (52) oder wendelartige Geometrie (58) aufweist und die Anschlagsoberfläche (18) an einem distalen Endbereich (14) der schlauchartigen (52) oder wendelartigen Geometrie (58) vorgesehen ist.

8. Längliches Implantationswerkzeug (10), umfassend:
- einen proximalen Abschnitt (12), der wenigstens abschnittsweise als Mandrin ausgebildet ist;
- einen distalen Abschnitt (14), der als flexibler Führungsdraht (44) ausgebildet ist; und
- ein statisches oder verstellbares Anschlagelement (16), das den proximalen und den distalen Abschnitt (12, 14) voneinander abgegrenzt und das eine dem distalen Abschnitt (14) zugewandte Anschlagoberfläche (18) umfasst.

9. Längliches Implantationswerkzeug (10) nach Anspruch 8, **dadurch gekennzeichnet, dass** das Anschlagelement (16) einen um eine Längsachse (20) des länglichen Implantationswerkzeugs (20) rotationssymmetrischen Bereich (22) umfasst, der quer zu der Längsachse (20) gegenüber dem distalen Abschnitt (14) einen größeren Querschnitt (22) aufweist.

10. Längliches Implantationswerkzeug (10) nach Anspruch 8, **dadurch gekennzeichnet, dass** das Anschlagelement (16) einen um eine Längsachse (20) rotations-asymmetrischen Bereich (32) umfasst, der quer zu der Längsachse (20) gegenüber dem distalen Abschnitt (14) einen erhöhten Querschnitt (34) aufweist.

11. Längliches Implantationswerkzeug (10) nach Anspruch 10, **dadurch gekennzeichnet, dass** das Anschlagelement (16) als Verdickung (38) des länglichen Implantationswerkzeugs (10) ausgebildet ist, die im Bereich der Anschlagoberfläche (18) eine quer zu der Längsachse (20) polygonale oder elliptische Querschnittsform aufweist.

12. Längliches Implantationswerkzeug (10) nach Anspruch 8, **dadurch gekennzeichnet, dass** es zweiteilig ausgeführt ist und einen als flexiblen Führungsdraht (44) ausgebildeten inneren Teil (40) sowie einen als Mandrin (46) ausgebildeten äußeren Teil (42) umfasst und der äußere Teil (42) mit einer inneren Mantelfläche (48) auf einer äußeren Mantelfläche (50) des inneren Teils (40) verlagerbar angeordnet ist, so dass der distale Abschnitt (14) von dem inneren Teil (40) gebildet wird und der proximale Abschnitt (12) von dem inneren Teil (40) und dem äußeren Teil (42) gebildet wird, wobei der äußere Teil (42) das Anschlagelement (16) umfasst.

13. Längliches Implantationswerkzeug (10) nach Anspruch 12, **dadurch gekennzeichnet, dass** der äußere Teil (42) eine schlauchartige (52) oder wendelartige Geometrie (58) aufweist und die Anschlagsoberfläche (18) an einem distalen Endbereich (54) der schlauchartigen (52) oder wendelartigen Geometrie (58) vorgesehen ist.

14. Implantierbare Elektrode (26), umfassend eine Elektrodenhülse (28), die einen Anschlagbereich (30) aufweist, der sich quer zu einer Längsachse (20) der Elektrodenhülse (28) erstreckt.

15. Implantierbare Elektrode (26) nach Anspruch 14, **dadurch gekennzeichnet, dass** der Anschlagbereich (30) einen polygonalen oder elliptischen Ausschnitt (36) aufweist.
